**Europäisches Patentamt**

**European Patent Office**

**Office européen des brevets**

(19)

(11) Veröffentlichungsnummer: **0 301 467 B1**

(12) ## EUROPÄISCHE PATENTSCHRIFT

(45) Veröffentlichungstag der Patentschrift: **02.01.92**

(21) Anmeldenummer: **88112001.8**

(22) Anmeldetag: **26.07.88**

(51) Int. Cl.⁵: **B65F 1/16**, B65D 43/06

(54) **Verschliessbarer Behälter zur Aufnahme von infektiösen Abfällen, Körperteilen und Organabfällen.**

(30) Priorität: **30.07.87 DE 8710452 U**

(43) Veröffentlichungstag der Anmeldung:
**01.02.89 Patentblatt 89/05**

(45) Bekanntmachung des Hinweises auf die
Patenterteilung:
**02.01.92 Patentblatt 92/01**

(84) Benannte Vertragsstaaten:
**AT BE CH DE FR GB IT LI LU NL**

(56) Entgegenhaltungen:
**EP-A- 0 267 776**
**US-A- 4 657 139**

(73) Patentinhaber: **ENTSORBIS ENTSORGUNGS-
BEHÄLTER VERTRIEB GMBH
Steinknappen 162
W-4330 Mülheim/Ruhr(DE)**

(72) Erfinder: **Lauterjung Pascal Peter
Beethovenstr. 279 a
W-5650 Solingen(DE)**

(74) Vertreter: **Sparing Röhl Henseler Patentanwälte European Patent Attorneys
Rethelstrasse 123
W-4000 Düsseldorf 1(DE)**

## Beschreibung

Die Erfindung betrifft einen verschließbaren Abfallbehälter zur Aufnahme von infektiösen Abfällen, Körperteilen und Organabfällen aus Krankenhäusern, Tierkliniken und sonstigen Einrichtungen des Gesundheitswesens mit einem Aufnahmegefäß und einem aufschnappbaren Deckel, dessen Deckelrand in formschlüssigen Eingriff mit einem oberen Rand des Aufnahmegefäßes bringbar ist.

Derartige Abfallbehälter dienen der Entsorgung von ansteckungsgefährlichen (infektiösen) Abfällen, Körperteilen und Organabfällen aus Krankenhäusern, Tierkliniken und sonstigen Einrichtungen des Gesundheitswesens, da diese Stoffe und Gegenstände besonderer Maßnahmen beim Sammeln, Transportieren und Beseitigen bedürfen, um Infektionen zu vermeiden. Diese Abfallbehälter dienen folglich der Verhütung und Bekämpfung von Krankenhausinfektionen. Auch der in Arztpraxen und medizinisch-mikrobiologischen Labors anfallende Müll besteht zumindest teilweise aus infektiösen Entsorgungsgütern, die einer Beseitigung durch Abfallbehälter bedürfen.

Gemäß der deutschen Vornorm DIN V 30 739 "Abfallbehälter für ansteckungsgefährliche Abfälle zur Verbrennung" müssen die für Mensch und Tier ansteckungsgefährlichen Abfälle in Einwegbehältern gesammelt und transportiert werden, die ungeöffnet mit den Abfällen verbrennbar sind. Dabei muß der Abfallbehälter undurchsichtig sein, desinfizierbar sein, nach ordnungsgemäßem Verschließen keimdicht sein, wasserdicht sein, im Gebrauchszustand formstabil und so befüllbar sein, daß mehrfache Eingabe zu unterschiedlichen Zeitpunkten bis zur höchstzulässigen Füllung möglich ist, durchdringfest sein, für die Dichte des Füllgutes von 0,3 kg/l ausgelegt sein, feuchtbeständig sein, mit Tragvorrichtungen versehen sein sowie standsicher, fallfest und innerhalb einer Bauart stapelbar sein. Nach der letzten Eingabe muß der Behälter außerdem für den Transport sicher verschließbar sein, wobei ein nachträgliches Öffnen visuell erkennbar sein muß. Schließlich muß der Deckel des Behälters für jede Eingabe so zu öffnen sein, daß man nicht zwangsweise mit dem Inhalt, d.h. mit den Innenseiten des Behälters, in Berührung kommt. Entsprechend dieser Norm hat das nutzbare Volumen 30l oder 60l zu betragen.

Zur Aufnahme infektiöser Stoffe und Gegenstände ist eine Vielzahl von Behältern bekannt, bei denen es sich um Behälter handelt, die der DIN V 30 739 vielfach nicht genügen. So sind einfache Eimer mit abnehmbarem Deckel zur Aufnahme infektiöser Abfälle bekannt, die weder keimdicht noch sicher verschließbar und damit zur Verwendung als Abfallbehälter für infektiösen Müll ungeeignet sind. Ferner sind derartige Abfallbehälter nicht schadstoffarm verbrennbar.

Wegen der Ansteckungsgefahr für Mensch und Tier sind auch Behälter bekannt, deren Deckel in formschlüssigem Eingriff mit einem Aufnahmegefäß stehen und auf diesem fest verriegelbar sind. Zur Verriegelung werden beispielsweise Aufschnappverschlüsse benutzt, die ein erneutes Öffnen des Deckels nach dem Aufsetzen verhindern. Diese bekannten Aufschnappverschlüsse sind allerdings aufwendig ausgestaltet und verlangen einen mit einem umlaufenden Haken ausgebildeten und stabilen Deckel, der zudem nicht keimdicht auf dem Aufnahmegefäß befestigbar ist. Das im allgemeinen zur Herstellung verbrennbarer Abfallbehälter verwendete Polyäthylen muß zur Erzielung der erforderlichen Festigkeit und Stabilität des Abfallbehälters, insbesondere des Deckels, gespritzt werden, wodurch die Herstellung derartiger Abfallbehälter teuer ist.

Aufgabe der Erfindung ist es daher, einen verschließbaren Abfallbehälter zur Aufnahme von infektiösen Abfällen, Körperteilen und Organabfällen nach dem Oberbegriff des Anspruchs 1 zu schaffen, der infektiöse Abfälle sicher aufnimmt und dabei einfach handhabbar und billig herstellbar ist.

Diese Aufgabe wird dadurch gelöst, daß der obere Rand des Aufnahmegefäßes jeweils entlang seiner Außen- und Innenseite eine Vielzahl benachbarter und mit Abstand zueinander angeordneter Nasen aufweist, der Deckelrand doppelwandig ausgebildet ist unter Einschluß eines Hohlraums für die formschlüssige Aufnahme des oberen Randes des Aufnahmegefäßes, und die Deckelrandaußenwandung eine Vielzahl von Durchbrüchen besitzt entsprechend der Anordnung der Nasen auf der Außenseite des oberen Randes des Aufnahmegefäßes.

Hierdurch wird erreicht, daß der Abfallbehälter nach dem Verschließen nicht mehr geöffnet werden kann, ohne daß ein nachträgliches Öffnen visuell erkennbar wäre. Der Deckel ermöglicht trotz seines geringen Gewichts ein keimdichtes Verschließen des Aufnahmegefäßes, da die Deckelrandinnenwandung dem Deckel Stabilität verleiht und außerdem von den Nasen an der Innenseite des Aufnahmegefäßes in den Behälter hineingebogen wird, so daß der Deckel unter einer Art Federkraft auf den Nasen der Außenseite des oberen Randes des Aufnahmegefäßes sitzt. Für ein leichteres Verschließen des Behälters können die Nasen ein abgerundetes längliches Profil aufweisen. Eine Erhöhung der Stabilität des oberen Randes des Aufnahmegefäßes kann durch eine abwechselnde Anordnung jeweils einer Nase auf der Innen- bzw. Außenseite des oberen Randes erreicht werden sowie durch Verstärkungsringe, die als Trag- und Stapelrand ausbildbar sind.

Der Abfallbehälter kann schließlich mit einem Traggriff in der Deckeloberseite oder einem Tragrand ausgebildet sein. Zur Unterstützung des keimdichten Verschließens kann auch ein Dichtungsring in den Deckelrand eingelassen werden und die Außenwandung des Deckels länger als die Innenwandung ausgebildet sein. Eine besonders schadstoffarme Verbrennung kann dadurch erreicht werden, daß der Abfallbehälter aus Polyäthylen blasbar ist. Für eine gute Stapelbarkeit der Abfallbehälter in Richtung einer Bodenfläche können die Seitenflächen verjüngt sein. Auch können die Nasen für ein einfacheres Einrasten ein abgerundetes Längsprofil besitzen, wobei zwischen zwei Nasen auf der Außenseite des oberen Randes des Aufnahmegefäßes jeweils eine Nase auf der Innenseite des oberen Randes angeordnet sein kann. Desweiteren können die Nasen jeweils eine Anstoßfläche und eine Gleitfläche umfassen, die einen spitzen Winkel einschließen, wobei die Anstoßflächen im wesentlichen parallel und mit Abstand zu einer oberen Kante des Randes angeordnet sind.

Schließlich kann der obere Rand über einen Anschlagrand an dem Aufnahmegefäß befestigt sein.

Weitere Ausgestaltungen der Erfindung sind der nachfolgenden Beschreibung und den Unteransprüchen zu entnehmen.

Die Erfindung wird nachstehend anhand des in den beigefügten Abbildungen dargestellten Ausführungsbeispiels näher erläutert.

Fig. 1     zeigt einen Abfallbehälter mit einem Aufnahmegefäß in einer Frontansicht und einem Deckel im Querschnitt.

Fig. 2     zeigt ein oberes Teilstück des Aufnahmegefäßes gemäß Fig. 1.

Fig. 3     zeigt eine Draufsicht des offenen Aufnahmegefäßes gemäß Fig. 1.

Fig. 4     zeigt eine Nase in einem Schnitt entlang A,B der Fig. 3.

Fig. 5     zeigt den Querschnitt des Deckels gemäß Fig. 1 vergrößert.

Fig. 6     zeigt teilweise geschnitten eine Frontansicht des Deckels.

Fig. 7     zeigt eine Draufsicht einer Deckelhälfte gemäß Fig. 1.

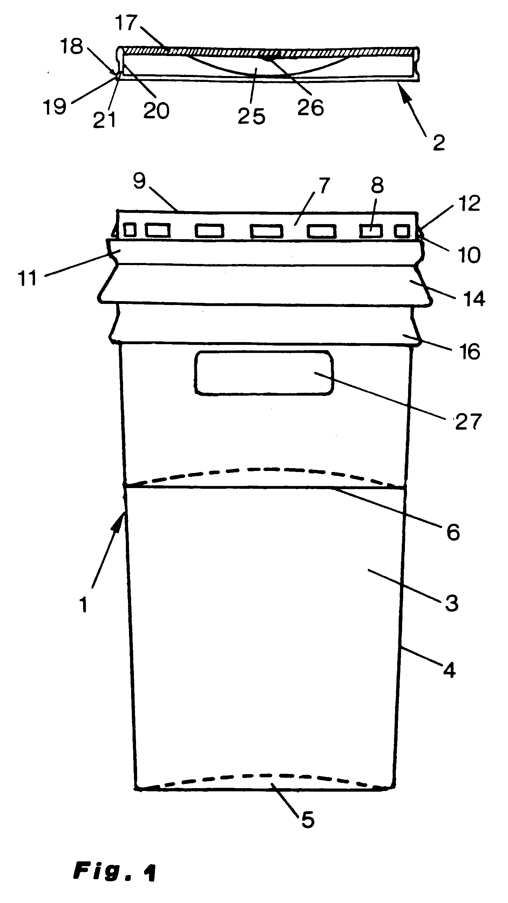

Der in Fig. 1 dargestellte verschließbare Abfallbehälter umfaßt ein Aufnahmegefäß 1 und einen Deckel 2, die einen Einwegbehälter aus Kunststoff bilden. Das Aufnahmegefäß 1 besteht aus einer Mülltonne 3, deren Umfangsfläche 4 sich leicht konisch zu einer Bodenfläche 5 verjüngt. Das nutzbare Volumen des Aufnahmegefäßes 1 beträgt 60 l oder 30 l, wobei ein 30 l Aufnahmegefäß eine Bodenfläche 6 aufweist, die das verfügbare Nutzvolumen der Mülltonne 3 auf 30 l verringert, so daß der Bereich der Mülltonne 3 zwischen der Bodenfläche 5 und 6 wegfallen kann, aber beide Größen des Aufnahmegefäßes 1 mit einer Form herstellbar sind. An der der Bodenfläche 5 gegenüberliegenden Seite weist das Aufnahmegefäß 1 einen oberen Rand 7 auf. Entlang seiner Außenseite besitzt dieser obere Rand 7 eine Vielzahl benachbarter und mit Abstand zueinander angeordneter erster Nasen 8, die an dem oberen Rand 7 umlaufend angeordnet sind. Vorzugsweise sind 8 bis 12 Nasen 8 vorgesehen.

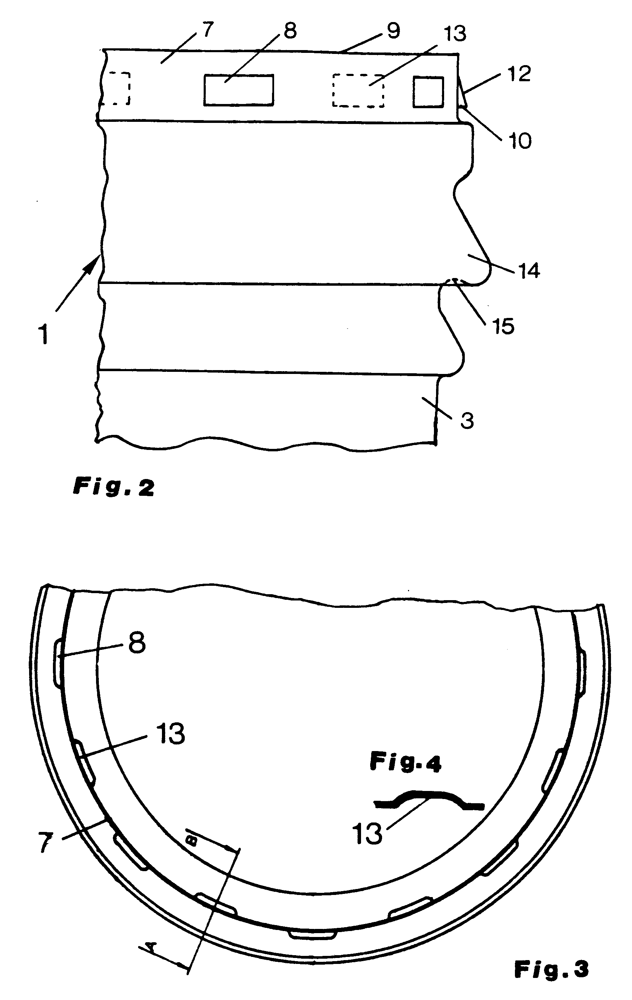

Wie insbesondere auch aus Fig. 2 erkennbar ist, weisen diese Nasen 8 jeweils eine zu einer oberen Kante 9 des oberen Randes 7 im wesentlichen parallele und mit Abstand angeordnete, vorspringende Arretierfläche 10 auf, benachbart zu denen die Anlagerand 11 für ein bündiges Aufsetzen des Deckels 2 an dem Aufnahmegefäß 1 befestigt ist. Dieser Anlagerand 11 weist dabei einen größeren Durchmesser als der obere Rand 7 auf. Neben der Arretierfläche 10 weisen die Nasen jeweils eine Gleitfläche 12 auf, die von der Außenseite des oberen Randes 7 vorspringt und mit der Arretierfläche 10 einen spitzen Winkel einschließt.

Gemäß Fig. 3 sind an der Innenseite des oberen Randes 7 benachbarte und mit Abstand zueinander angeordnete zweite Nasen 13 vorgesehen, die entsprechend den Nasen 8 ausgebildet sein können, nach dem dargestellten Ausführungsbeispiel aber ein Längsprofil gemäß Fig. 4 aufweisen. Danach bestehen die Nasen 13 aus einer abgerundet vorspringenden Fläche, die abgerundet wieder zur Innenseite des oberen Randes 7 zurückspringt. Die Nasen 13 erstrecken sich dabei weniger weit in den Innenraum des Aufnahmegefäßes 1 als die Nasen 8 von der Außenseite des oberen Randes 7. Vorzugsweise sind diese Nasen 13 jeweils zwischen zwei Nasen 8 symmetrisch angeordnet, wodurch die Stabilität des oberen Randes 7 erhöht wird. Fig. 3 zeigt darüber hinaus, daß die Nasen 8 und 13 jeweils ein abgerundetes Querprofil besitzen.

Unterhalb des oberen Randes 7 weist das Aufnahmegefäß 1 noch Verstärkungsringe auf, von denen einer als Tragrand 14 ausgebildet ist, der gegenüber den Seitenflächen 3 vorspringt und mit einer umlaufenden Griffmulde 15 versehen ist. Benachbart zu diesem Tragrand 14 ist ein zweiter Verstärkungsring angeordnet, der als vorspringender Stapelrand 16 ausgebildet ist Die Verstärkungsringe 14 und 15 geben dem Aufnahmegefäß eine hohe Stabilität, so daß dieses trotz geringen Gewichts blasbar ist. Durch die konische Ausführung der Umfangsfläche 4 des Aufnahmegefäßes können die leeren Aufnahmegefäße jeweils ineinander gestapelt werden, wobei die obere Kante 9 an den Stapelrand 16 eines darüber gestapelten Aufnahmegefäßes stößt. Durch den Tragrand 14 lassen sich die einmal gestapelten Aufnahmegefäße wieder leicht voneinander trennen.

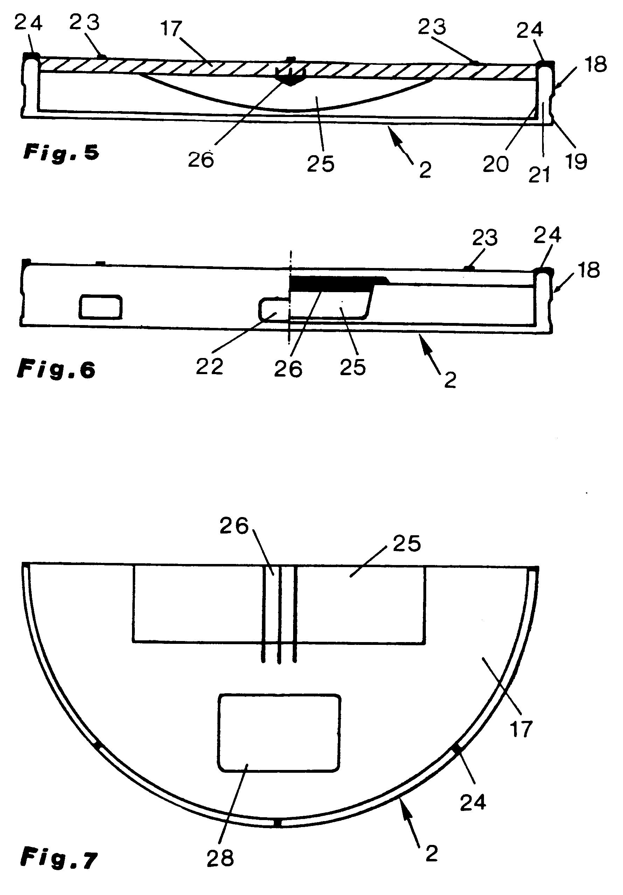

Wie insbesondere Fig. 1 und Fig. 5 zeigen, umfaßt der Deckel 2 eine Dekkeloberseite 17 und einen Deckelrand 18. Der Deckelrand 18 besteht aus einer umlaufenden Außenwandung 19 und einer umlaufenden Innenwandung 20, die gegenüber der Deckeloberseite 17 vorspringen. Die Innenwandung 20 und die Außenwandung 20 erstrecken sich von der Deckeloberseite 17 unter Einschluß eines Hohlraumes 21, indem die Wandungen 19 und 20 parallel und mit Abstand zueinander angeordnet sind. Vorzugsweise überlappt die Außenwandung 19 die Innenwandung 20, so daß die Innenwandung 20 kürzer ist. In der Außenwandung 19 befinden sich Durchbrüche 22, die benachbart und mit Abstand zueinander und dabei umlaufend entlang des Deckelrandes 18 angeordnet sind. Die Durchbrüche 22 sind entsprechend dem Abstand der Nasen 8 auf der Außenseite des oberen Randes 7 angeordnet, wobei die Abmessungen der Durchbrüche so gewählt sind, daß die Nasen 8 bei aufgesetztem Deckel 2 in die Durchbrüche 22 einschnappbar sind. Der Hohlraum 21 besitzt Abmessungen gemäß dem oberen Rand 7, um diesen formschlüssig aufnehmen zu können. Zur Kennzeichnung des Orts der Durchbrüche 22 im Deckelrand 18 ist über jedem Durchbruch 22 ein Erkennungsnocken 23 angeordnet. Auf der Deckeloberseite 17 ist weiterhin eine umlaufende Stapelkante 24 angeordnet, um gegebenenfalls auch die gefüllten Abfallbehälter stapeln zu können. In die Deckeloberseite 17 ist eine Traggriffmulde 25 eingelassen, über der ein Traggriff 26 angeordnet ist.

Zum Verschließen des Aufnahmegefäßes 1 durch den Deckel 2 wird der ebenfalls aus Kunststoff bestehende Deckel 2 derart auf das Aufnahmegefäß 1 gesetzt, daß der obere Rand 7 in den Hohlraum 21 des Deckelrandes 18 gleitet. Durch die Elastizität der Außenwandung 19 biegt sich diese bei Aufdrücken des Deckels 2 auf das Aufnahmegefäß 1 etwas nach außen, wodurch die Nasen 8 durch die Durchbrüche 22 treten können. Die Nasen 13 an der Innenwandung 20, die ebenfalls durch ihre im wesentlichen dünnwandige Ausbildung elastisch ist, bewirken ein Biegen der Innenwandung 20 in den Innenbereich des Aufnahmegefäßes 1, wodurch ein Druck auf den Deckel 2 entgegen der Aufsetzrichtung ausgeübt wird. Hierdurch wird der Deckel 2 mit den Unterkanten der Durchbrüche 22 gegen die Arretierflächen 10 der Nasen 8 gedrückt. Ein erneutes Öffnen des Deckel 2 nach Verschließen des Abfallbehälters kann daher nur durch teilweise Zerstörung des Deckels 2 erreicht werden. Das Aufnahmegefäß 1 und der Deckel 2 sind somit über einen elastischen Schnappverschluß aneinander befestigbar, der ein erneutes Öffnen des Abfallbehälters verhindert. Zur Unterstützung eines keimdichten Verschlusses des Abfallbehälters kann in dem Hohlraum 21 des Deckelrandes 18 ein nicht dargestellter Dichtungsring angeordnet sein, der mit der Kante 9 des oberen Randes 7 in Eingriff bringbar ist, um den Abfallbehälter sicher zu verschließen. Zur Erzielung einer schadstoffarmen Verbrennung besteht der Abfallbehälter vorzugsweise aus Polyäthylen Der Abfallbehälter ist zudem blasbar, da Aufnahmegefäß 1 und Deckel 2 trotz geringen Gewichts eine hohe Stabilität besitzen.

Der vorstehend beschriebene Abfallbehälter erfüllt somit nicht nur die Anforderung der Norm DIN V 30 739, indem er ferner auch mit den erforderlichen Beschriftungsetiketten 27 und 28 ausgestattet werden kann, sondern ist durch seine Blasbarkeit auch billig herstellbar.

**Patentansprüche**

1. Verschließbarer Abfallbehälter zur Aufnahme von infektiösen Abfällen, Körperteilen und Organabfällen aus Krankenhäusern, Tierkliniken und sonstigen Einrichtungen des Gesundheitswesens mit einem Aufnahmegefäß (1) und einem aufschnappbaren Deckel (2), dessen Deckelrand (18) in formschlüssigen Eingriff mit einem oberen Rand (7) des Aufnahmegefäßes (1) bringbar ist, **dadurch gekennzeichnet**, daß der obere Rand (7) des Aufnahmegefäßes (1) jeweils entlang seiner Außen- und Innenseite eine Vielzahl benachbarter und mit Abstand zueinander angeordneter Nasen (8, 13) aufweist, der Deckelrand (18) doppelwandig ausgebildet ist unter Einschluß eines Hohlraums (21) für die formschlüssige Aufnahme des oberen Randes (7) des Aufnahmegefäßes (1), und die Deckelrandaußenwandung (19) eine Vielzahl von Durchbrüchen (22) besitzt entsprechend der Anordnung der Nasen (8) auf der Außenseite des oberen Randes (7) des Aufnahmegefäßes (1).

2. Verschließbarer Abfallbehälter nach Anspruch 1, dadurch gekennzeichnet, daß die Nasen (8, 13) ein abgerundetes Längsprofil besitzen.

3. Verschließbarer Abfallbehälter nach Anspruch 1 oder 2, dadurch gekennzeichnet, daß zwischen zwei Nasen (8) auf der Außenseite des oberen Randes (7) des Aufnahmegefäßes (1) jeweils eine Nase (13) auf der Innenseite des oberen Randes (7) angeordnet ist.

4. Verschließbarer Behälter nach einem der Ansprüche 1 bis 3, dadurch gekennzeichnet, daß die Nasen (8) jeweils eine Anstoßfläche (10) und eine Gleitfläche (12) umfassen, die einen spitzen Winkel einschließen, wobei die Anstoßflächen (10) im wesentlichen parallel und mit Abstand zu einer oberen Kante (9) des oberen Randes (7) angeordnet sind.

5. Verschließbarer Abfallbehälter nach einem der Ansprüche 1 bis 4, dadurch gekennzeichnet, daß der obere Rand (7) über einen Anschlagrand (11) an dem Aufnahmegefäß (1) befestigt ist.

6. Verschließbarer Abfallbehälter nach einem der Ansprüche 1 bis 5, dadurch gekennzeichnet, daß das Aufnahmegefäß (1) einen Tragrand (14) und benachbart dazu einen Stapelrand (16) aufweist.

7. Verschließbarer Abfallbehälter nach einem der Ansprüche 1 bis 6, dadurch gekennzeichnet, daß in dem Hohlraum (21) ein Dichtungsring angeordnet ist.

8. Verschließbarer Abfallbehalter nach einem der Ansprüche 1 bis 7, dadurch gekennzeichnet, daß der Deckel (2) eine Ausnehmung (25) für einen Tragegriff (26) enthält.

9. Verschließbarer Abfallbehälter nach einem der Ansprüche 1 bis 8, dadurch gekennzeichnet, daß das Aufnahmegefäß (1) stapelbare in Richtung einer Bodenfläche (5) verjüngte Seitenflächen (4) aufweist.

10. Verschließbarer Abfallbehälter nach einem der Ansprüche 1 bis 9, dadurch gekennzeichnet, daß das Aufnahmegefäß (1) und der Deckel (2) jeweils einstückig ausgebildet sind und aus Polyäthylen bestehen.

**Claims**

1. Sealable refuse container for receiving infectious refuse, body parts and organic refuse from hospitals, veterinary clinics, and other health care institutions, with a receptacle (1) and a snap-on lid (2), the lid rim (18) of which can be brought into positive engagement with an upper rim (7) of the receptacle (1), characterized in that the upper rim (7) of the receptacle (1) has a plurality of adjacent projections (8, 13) arranged at intervals from one another along each of its outer and inner sides, the lid rim (18) is of double-walled construction and encloses a cavity (21) for the positive engagement of the upper rim (7) of the receptacle (1), and the outer wall (19) of the lid rim has a plurality of openings (22) corresponding to the arrangement of the projections (8) on the outside of the upper rim (7) of the receptacle (1).

2. A sealable refuse container according to claim 1, characterized in that the projections (8, 13) have a rounded elongate profile.

3. A sealable refuse container according to claim 1 or 2, characterized in that between two projections (8) on the outside of the upper rim (7) of the receptacle (1) there is arranged one projection (13) on the inside of the upper rim (7).

4. A sealable container according to one of claims 1 to 3, characterized in that the projections (8) each comprise an abutting face (10) and a slide face (12) which form an acute angle, the abutting faces (10) being arranged substantially parallel and at a distance from an upper edge (9) of the upper rim (7).

5. A sealable refuse container according to one of claims 1 to 4, characterized in that the upper rim (7) is fastened to the receptacle (1) by way of an abutment edge (11).

6. A sealable refuse container according to one of claims 1 to 5, characterized in that the receptacle (1) has a carrying lip (14) and adjacent thereto a stacking lip (16).

7. A sealable refuse container according to one of claims 1 to 6, characterized in that a sealing ring is arranged in the cavity (21).

8. A sealable refuse container according to one of claims 1 to 7, characterized in that the lid (2) contains a recess (25) for a carrying handle (26).

9. A sealable refuse container according to one of claims 1 to 8, characterized in that the receptacle (1) has stackable lateral faces (4) that taper towards a bottom face (5).

10. A sealable refuse container according to one of claims 1 to 9, characterized in that the receptacle (1) and the lid (2) are each of integral construction and consist of polyethylene.

**Revendications**

1. Récipient à déchets obturable destiné à recevoir des déchets infectieux, des parties corporelles et des déchets organiques provenant d'hôpitaux, de cliniques vétérinaires et autres installations du domaine sanitaire, comportant un réceptacle (1) et un couvercle (2) ouvrable par articulation dont le bord (18) peut être amené en prise avec un bord supérieur (7) du réceptacle (1), caractérisé par le fait que le bord supérieur (7) du réceptacle (1) présente, respectivement le long de son côté extérieur et de son côté intérieur, une pluralité d'ergots (8,13) disposés à de faibles intervalles les uns des autres, le bord du couvercle (18) étant à double paroi et délimitant un espace creux (21) permettant de recevoir de manière solidaire le bord supérieur (7) du réceptacle (1), la paroi extérieure (19) du bord du couvercle étant munie d'une pluralité de fentes (22) dont la disposition correspond à celle des ergots (8) placés sur le côté extérieur du bord supérieur (7) du réceptacle (1).

2. Récipient à déchets obturable selon la revendication 1, caractérisé par le fait que les ergots (8,13) présentent un profil longitudinal arrondi.

3. Récipient à déchets obturable selon la revendication 1 ou 2, caractérisé par le fait qu'entre deux ergots (8) sur le côté extérieur du bord supérieur (7) du réceptacle (1) est disposé en correspondance un ergot (13) sur le côté intérieur du bord supérieur (7).

4. Récipient obturable selon l'une des revendications 1 à 3, caractérisé par le fait que les ergots (8) comportent respectivement une face de butée (10) et une face de glissement (12) qui forment entre elles un angle aigu, les faces de butée (10) étant disposées sensiblement parallèlement et à une certaine distance de l'une des arêtes supérieures (9) du bord supérieur (7);

5. Récipient à déchets obturable selon l'une des revendications 1 à 4, caractérisé par le fait que le bord supérieur (7) est relié au réceptacle (1) par un rebord de butée (11).

6. Récipient à déchets obturable selon l'une des revendications 1 à 5, caractérisé par le fait que le réceptacle (1) présente un rebord de portage (14) et au voisinage de celui-ci un rebord de gerbage (16).

7. Récipient à déchets obturable selon l'une des revendications 1 à 6, caractérisé par le fait que dans l'espace creux (21) est disposé un joint d'étanchéité.

8. Récipient à déchets obturable selon l'une des revendications 1 à 7, caractérisé par le fait que le couvercle (2) supporte un évidement (25) pour une poignée de transport (26).

9. Récipient à déchets obturable selon l'une des revendications 1 à 8, caractérisé par le fait que le réceptacle (1) présente des faces latérales (4) formant un tronc de cône en direction du fond (5) pour permettre le gerbage.

10. Récipient à déchets obturable selon l'une des rebvendications 1 à 9, caractérisé par le fait que le réceptacle (1) et le couvercle (2) sont réalisée respectivement en une seule pièce et constitués en polyéthylène.

**Fig. 1**

**Fig.2**

**Fig.4**

**Fig.3**

Fig.5

Fig.6

Fig.7